(19) Europäisches Patentamt
European Patent Office
Office européen des brevets

(11) Veröffentlichungsnummer: **0 066 226**
**A1**

(12) **EUROPÄISCHE PATENTANMELDUNG**

(21) Anmeldenummer: 82104501.0

(22) Anmeldetag: 24.05.82

(51) Int. Cl.³: G 03 C 7/36
G 03 C 7/30, G 03 C 7/26
G 03 C 7/00

(30) Priorität: 03.06.81 DE 3121955

(43) Veröffentlichungstag der Anmeldung: 08.12.82 Patentblatt 82/49

(84) Benannte Vertragsstaaten: BE DE FR GB

(71) Anmelder: AGFA-GEVAERT Aktiengesellschaft
D-5090 Leverkusen 1(DE)

(72) Erfinder: Wolff, Erich, Dr.
Balkhauser Weg 6
D-5650 Solingen(DE)

(72) Erfinder: Lowski, Dieter
Berliner Ring 5
D-5150 Bergheim Erft(DE)

(54) **Chromogenes Entwicklungsverfahren zur Herstellung eines Purpurbildes und hierfür geeignetes farbfotografisches Aufzeichnungsmaterial.**

(57) Purpurbilder werden hergestellt durch chromogene Entwicklung eines farbfotografischen Aufzeichnungsmaterials in Gegenwart eines Farbkupplers der Formel

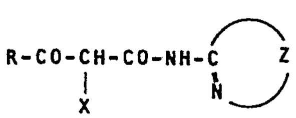

worin bedeuten
Z einen Rest zur Vervollständigung eines heterocyclischen Ringes,
X eine Fluchtgruppe,
R Alkyl, Alkoxy, Aryl oder eine heterocyclische Gruppe.
Die Kuppler sind gegen Formalin stabil und bilden primär Gelbfarbstoffe, die durch spontane Cyclisierung in Purpurfarbstoffe übergehen.

EP 0 066 226 A1

AGFA-GEVAERT
AKTIENGESELLSCHAFT
Patentabteilung

5090 Leverkusen, Bayerwerk
Hs/kl-c

# Chromogenes Entwicklungsverfahren zur Herstellung eines Purpurbildes und hierfür geeignetes farbfotografisches Aufzeichnungsmaterial

Die Erfindung betrifft ein chromogenes Entwicklungsverfahren zur Herstellung eines Purpurbildes unter Verwendung spezieller Acylacetamidoverbindungen, sowie ein hierfür geeignetes farbfotografisches Aufzeichnungsmaterial.

Es ist bekannt, farbfotografische Bilder durch chromogene Entwicklung herzustellen, d.h. dadurch, daß man ein bildmäßig belichtetes Aufzeichnungsmaterial mit mindestens einer Silberhalogenidemulsionsschicht in Gegenwart geeigneter Farbkuppler mittels geeigneter farbbildender Entwicklersubstanzen - sogenannter Farbentwickler - entwickelt, wobei das in Übereinstimmung mit dem Silberbild entstehende Oxidationsprodukt der Entwicklersubstanzen mit dem Farbkuppler unter Bildung eines Farbstoffbildes reagiert. Als Farbentwickler werden gewöhnlich aromatische, primäre Aminogruppen enthaltende Verbindungen, insbesondere solche vom p-Phenylendiamintyp, verwendet.

An die Farbkuppler sowie an die daraus durch chromogene Entwicklung erhaltenen Farbstoffe werden in der Praxis eine Reihe von Forderungen gestellt. So soll die Kupplungsgeschwindigkeit der Farbkuppler mit dem Oxidationsprodukt des Farbentwicklers möglichst groß sein. Die Farbkuppler sowie die daraus erhaltenen Farbstoffe müssen hinreichend stabil sein gegenüber Licht, erhöhter Temperatur und Feuchtigkeit. Dies gilt sowohl für frisches Material, als auch für verarbeitetes Material. Beispielsweise darf der in den Bildweißen des verarbeiteten Materials noch vorhandene Restkuppler nicht vergilben. Außerdem sollen die Farbstoffe hinreichend beständig sein gegenüber gasförmigen reduzierenden oder oxidierenden Agentien. Sie müssen ferner diffusionsfest in der Bildschicht verankert sein und sollen sich bei der chromogenen Entwicklung als möglichst feines Korn abscheiden. Weiterhin dürfen die mechanischen Eigenschaften der Schichten durch die Farbkuppler nicht beeinträchtigt werden. Schließlich müssen die aus den Farbkupplern bei der chromogenen Entwicklung entstehenden Farbstoffe eine günstige Absorptionskurve aufweisen mit einem Maximum, das der Farbe des jeweils gewünschten Teilbildes entspricht, und möglichst geringen Nebenabsorptionen. So soll im Idealfall ein Purpurfarbstoff grünes Licht nahezu vollständig absorbieren und blaues sowie rotes Licht weitgehend durchlassen.

Als Purpurkuppler, d.h. als Farbkuppler, die zur Erzeugung des purpurnen Farbbildes geeignet sind, werden im allgemeinen Verbindungen verwendet, die sich vom 2-Pyrazolon-5 ableiten.

Bei Verwendung derartiger Pyrazolonkuppler stellt sich in zunehmendem Maße deren Reaktivität mit Formalin als Nachteil heraus. Durch Reaktion mit Formalin, das in Spuren in der Atmosphäre vorhanden ist und insbesondere von Holz oder Kunststoffen, wie sie bei der Möbelherstellung verwendet werden, über längere Zeit freigesetzt wird, wird die Kupplungsstelle dieser Kuppler blockiert und hierdurch Empfindlichkeit und maximale Farbdichte der farbfotografischen Aufzeichnungsmaterialien nachteilig beeinflußt. Dieser Effekt wirkt sich besonders gravierend aus, wenn ein farbfotografisches Aufzeichnungsmaterial mit solchen Farbkupplern belichtet oder unbelichtet längere Zeit in einer formalinhaltigen Umgebung, z.B. in neueren Kunststoffschränken aufbewahrt wird, bevor es zur Entwicklung gelangt.

Zur Behebung dieses Nachteils sind schon verschiedene Vorschläge gemacht worden. Die bekannten Lösungsansätze beruhen entweder darauf, daß den Aufzeichnungsmaterialien sogenannte Formalinfänger zugesetzt werden, oder darauf, daß die Reaktivität der Kuppler in der einen oder anderen Weise reduziert wird, z.B. dadurch, daß anstelle der üblichen 4-Äquivalentkuppler die entsprechenden 2-Äquivalentkuppler verwendet werden, daß die Kuppler in der Enolform mit durch Acylierung blockierter enolischer Hydroxylgruppe verwendet werden, oder daß die Kuppler in der Enolform als Fluchtgruppe an die Kupplungsstelle eines anderen Kupplers gebunden sind, der bei der Entwicklung farblose oder alkalilösliche Produkte ergibt (Research Disclosure 19 536; Juli 1980). Es liegt auf der Hand, daß bei allen diesen Methoden eine Verbesserung der Stabilität gegen Formalin mit anderen Nachteilen erkauft wird, z.B. einer erhöhten Schichtbelastung oder einer reduzierten Empfindlichkeit der Kuppler. Bei der zuletzt genannten Methode kommt hinzu, daß es sich bei den dort verwendeten Kupplern um 6-Äquivalentkuppler handelt; zwei Oxidationsäquivalente werden verbraucht um den Purpurkuppler als Fluchtgruppe von dem anderen Kuppler freizusetzen und dadurch zu aktivieren, und für die eigentliche Farbkupplung werden weitere vier Oxidationsäquivalente benötigt. Dies macht einen erhöhten Silberauftrag erforderlich und ist allein schon aus diesem Grund als Nachteil anzusehen.

Es ist bekannt, daß 2-Acetoacetamidopyridin bei der chromogenen Entwicklung zunächst wie erwartet einen gelben Farbstoff bildet, der hernach in einen Purpurfarbstoff übergeht /J. Amer, Chem. Soc. 66, 1805 (1944)/. Diese Reaktion erfordert insgesamt sechs Oxidationsäquivalente und hat wohl nicht zuletzt aus diesem Grund für die Praxis keine Bedeutung erlangt.

Es ist bereits bekannt, bei Farbkupplern, insbesondere bei solchen des offenkettigen Ketomethylentyps, d.h.

bei Gelbkupplern die Menge des für die Farbkupplung erforderlichen Silberhalogenids dadurch zu reduzieren, daß anstelle der üblichen 4-Äquivalentkuppler die entsprechenden 2-Äquivalentkuppler verwendet werden, die in der Kupplungsstelle mit einer bei der Farbkupplung abspaltbaren Gruppe (Fluchtgruppe) substituiert sind.

Der Erfindung liegt die Aufgabe zugrunde, ein chromogenes Entwicklungsverfahren zur Herstellung von Purpurbildern anzugeben, bei dem Kuppler verwendet werden, die ausreichend reaktiv und gleichzeitig stabil gegen Formalin sind und dabei keinen erhöhten Silberauftrag erfordern.

Gegenstand der Erfindung ist ein Verfahren zur Herstellung eines Purpurbildes durch chromogene Entwicklung, bei dem ein bildmäßig belichtetes farbfotografisches Aufzeichnungsmaterial mit mindestens einer Silberhalogenidemulsionsschicht in Gegenwart einer Acylacetamidoverbindung und einer eine primäre Aminogruppe enthaltenden Farbentwicklerverbindung entwickelt wird, dadurch gekennzeichnet, daß die Entwicklung in Gegenwart einer Verbindung der folgenden Formel I durchgeführt wird

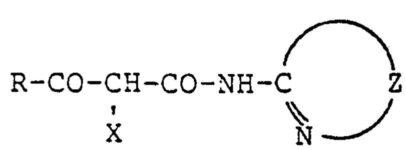

I

worin bedeuten

Z einen Rest zur Vervollständigung eines mindestens ein Stickstoffatom enthaltenden heterocyclischen Ringes, insbesondere eines 6-gliedrigen Ringes

X eine bei der chromogenen Entwicklung abspaltbare Gruppe des für 2-Äquivalentgelbkuppler üblichen Typs,

R Alkyl, z.B. Methyl, Trifluormethyl, Isopropyl, tert.-Butyl, n-Hexyl, n-$C_{13}H_{27}$, Benzyl, Alkoxy, z.B. Methoxy, Ethoxy, Aryl, substituiertes Aryl, z.B. Phenyl, p-Methoxyphenyl oder eine heterocyclisch aromatische Gruppe, z.B. Thienyl.

Der durch Z vervollständigte heterocyclische Ring ist vorzugsweise aromatischer Natur und kann gegebenenfalls außer dem in der Formel dargestellten Stickstoffatom weitere Stickstoffatome und/oder ankondensierte Ringe z.B. einen ankondensierten Benzolring enthalten. Beispiele für derartig vervollständigte heterocyclische Ringe sind die heterocyclischen Ringe von Pyridin, 1,3-Diazin, 1,3,5-Triazin, Chinolin und Isochinolin.

Bei der durch X dargestellten abspaltbaren Gruppe handelt es sich um ein Halogenatom (z.B. F, Cl, Br, I) oder um eine organische Gruppe, die in der Regel über ein Sauerstoff-, Schwefel- oder Stickstoffatom an die Kupplungsstelle des Kupplermoleküls angeknüpft ist. Falls es sich bei der abspaltbaren Gruppe um eine cyclische Gruppe handelt, kann die Anknüpfung an die

Kupplungsstelle des Kupplermoleküls entweder direkt über ein Atom, das Bestandteil eines Ringes ist, z.B. ein Stickstoffatom, oder indirekt über ein zwischengeschaltetes Bindeglied erfolgt sein. Derartige abspaltbare Gruppen sind in großer Zahl bekannt, z.B. als Fluchtgruppen von 2-Äquivalentgelbkupplern.

Beispiele von über Sauerstoff angeknüpften abspaltbaren Gruppen entsprechen der Formel

$$-O-R^1,$$

worin $R^1$ für einen acyclischen oder cyclischen organischen Rest steht, z.B. für Alkyl, Aryl, eine heterocyclische Gruppe oder Acyl, das sich beispielsweise ableitet von einer organischen Carbon- oder Sulfonsäure. Bei besonders bevorzugten abspaltbaren Gruppen dieser Art bedeutet $R^1$ eine gegebenenfalls substituierte Phenylgruppe.

Beispiele von über Stickstoff angeknüpften abspaltbaren Gruppen sind in den folgenden deutschen Offenlegungsschriften (DE-OS) beschrieben:

2 057 941, 2 163 812, 2 213 461, 2 219 917, 2 261 361, 2 263 875, 2 318 807, 2 329 587, 2 344 155, 2 363 675, 2 433 812, 2 441 779, 2 442 703, 2 528 638, 2 528 860, 2 637 817, 2 818 373, 3 020 416.

Hierbei handelt es sich durchweg um 5- oder 6-gliedrige heterocyclische Ringe, die über ein Ringstickstoffatom mit der Kupplungsstelle des offenkettigen Ketomethylenkupplers verbunden sind. Die heterocyclischen Ringe enthalten vielfach benachbart zu dem die Bindung an das Kupplermolekül vermittelnden Stickstoffatom aktivierende Gruppen, z.B. Carbonyl- oder Sulfonylgruppen oder Doppelbindungen.

Wenn die abspaltbare Gruppe über ein Schwefelatom an die Kupplungsstelle des Kupplers gebunden ist, kann es sich bei ihr um den Rest einer diffusionsfähigen Mercaptoverbindung handeln, die die Entwicklung von Silberhalogenid zu inhibieren vermag. Derartige Inhibitorreste sind vielfach als an die Kupplungsstelle von Kupplern, auch offenkettigen Ketomethylenkupplern gebundene abspaltbare Gruppe beschrieben worden, z.B. in US 3 227 554.

Bei der chromogenen Entwicklung von 2-Acetoacetamidopyridin erfolgt zunächst wie bei 4-Äquivalentgelbkupplern die Bildung eines gelben Azomethinfarbstoffes unter Verbrauch von 4 Oxidationsäquivalenten. Der gelbe Azomethinfarbstoff unterliegt jedoch unter den Reaktionsbedingungen einer spontanen Cyclisierung wobei zwischen dem Kohlenstoffatom der Azomethingruppe und dem Stickstoffatom des Pyridinringes eine Bindung gebildet wird unter Abspaltung der Acetylgruppe. Zur Oxidation des hierbei gebildeten Leukofarbstoffes zum Purpurfarbstoff werden weitere 2 Oxidationsäquivalente verbraucht. Der Purpurfarbstoff ist ein vom

3a-Azaindolon-2-ringsystem abgeleiteter Azomethinfarbstoff.

Die erfindungsgemäß verwendeten Farbkuppler (z.B. R = Methyl; Z vervollständigt einen Pyridinring) unterscheiden sich von dem bekannten 2-Acetoacetamidopyridin im wesentlichen dadurch, daß sie in der Kupplungsstelle eine (von Wasserstoff verschiedene) abspaltbare Gruppe X enthalten. Diese abspaltbare Gruppe wird bei der chromogenen Entwicklung eliminiert, ohne daß für diesen Teilschritt 2 Oxidationsäquivalente verbraucht werden. Somit werden insgesamt nur 4 Oxidationsäquivalente benötigt, so daß der Nachteil des erhöhten Silberverbrauches gegenüber den üblichen von Pyrazolon abgeleiteten 4-Äquivalentkupplern entfällt.

Über die Struktur der Gruppe R ist es möglich die Austrittstendenz der Acylgruppe R-CO- und damit die Ringbildung in weiten Grenzen (z.B. pH-Bereichen) je nach Wunsch zu variieren. So liefert R = Methyl ausschließlich den Purpurfarbstoff, während bei R = tert.-Butyl unter den gleichen Bedingungen die Ringbildung nur unvollständig erfolgt, so daß neben dem Purpurfarbstoff noch der primär gebildete Gelbfarbstoff vorliegt. In gleicher Weise läßt sich das Ausmaß der Ringschlußreaktion durch Substitution mit geeigneten Substituenten S beeinflussen, wenn R eine Gruppe 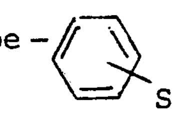 bedeutet.

Die Einführung von Ballastgruppen, die zur diffusionsfesten Einlagerung in farbfotografische Aufzeichnungsmaterialien erforderlich ist, kann in der verschiedensten Weise vorgenommen werden, z.B. über R, X oder den durch Z vervollständigten heterocyclischen Ring. Da R (als R-CO) und X bei der chromogenen Entwicklung eliminiert werden und daher im gebildeten Farbstoff nicht mehr vorhanden sind, wirkt eine Ballastierung über R oder X nur temporär für die Kuppler; der gebildete Farbstoff kann diffusionsfähig sein, oder ebenfalls diffusionsfest, wenn ballastgruppenhaltige Farbentwicklerverbindungen verwendet werden, wie sie etwa in DE-OS 1 931 057 beschrieben sind. Wenn die Farbkuppler permanent mit Ballastgruppen versehen werden sollen, so daß die Ballastgruppe auch im gebildeten Purpurfarbstoff noch vorhanden ist, gelingt dies über den durch Z vervollständigten heterocyclischen Ring. Derartige Kuppler entsprechen der Formel II

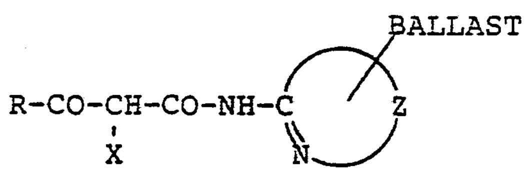

(II)

worin R, X und Z die bereits angegebene Bedeutung haben und BALLAST einen diffusionsfestmachenden Rest bedeutet.

Als diffusionsfestmachende Reste sind solche Reste anzusehen, die es ermöglichen, die erfindungsgemäßen

Farbkuppler in den üblicherweise bei fotografischen Materialien verwendeten hydrophilen Kolloiden diffusionsfest einzulagern. Hierzu sind vorzugsweise organische Reste geeignet, die im allgemeinen geradkettige oder verzweigte aliphatische Gruppen mit im allgemeinen 8 bis 20 C-Atomen und gegebenenfalls auch carbocyclische oder heterocyclische gegebenenfalls aromatische Gruppen enthalten. Mit dem übrigen Molekülteil sind diese Reste entweder direkt oder indirekt, z.B. über eine der folgenden Gruppen verbunden: $-NHCO-$, $-NHSO_2-$, $-NR-$, wobei R Wasserstoff oder Alkyl bedeutet, -O- oder -S-. Zusätzlich kann der diffusionsfestmachende Rest auch wasserlöslichmachende Gruppen enthalten, wie z.B. Sulfogruppen oder Carboxylgruppen, die auch in anionischer Form vorliegen können. Da die Diffusionsfestigkeit von der Molekülgröße der verwendeten Gesamtverbindung abhängt, genügt es in bestimmten Fällen, z.B., wenn das verwendete Gesamtmolekül groß genug ist, als "diffusionsfestmachende Reste" auch kürzerkettige Reste zu verwenden.

Bei Wahl geeigneter Farbentwicklerverbindungen ist es jedoch auch möglich erfindungsgemäße Farbkuppler zu verwenden, die weder über R noch über X noch über den durch Z vervollständigten heterocyclischen Ring eine echte Ballastgruppe enthalten, z.B. wenn die Kuppler im sogenannten Einentwicklungsverfahren verwendet werden.

Beispiele geeigneter Purpurkuppler gemäß der Erfindung sind im folgenden angegeben:

1 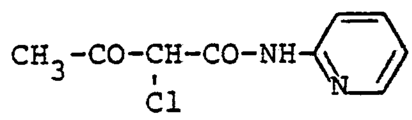

2 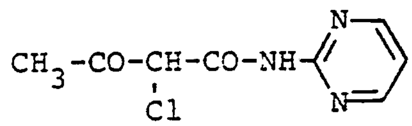

3 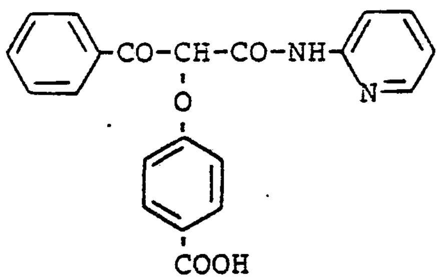

4 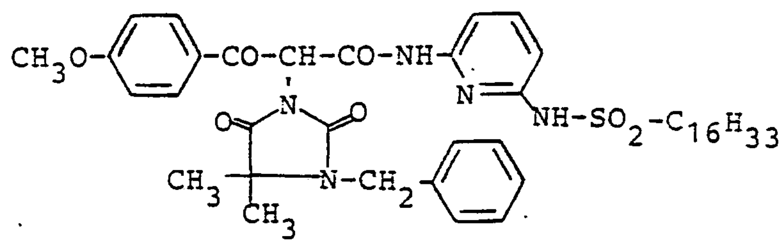

5 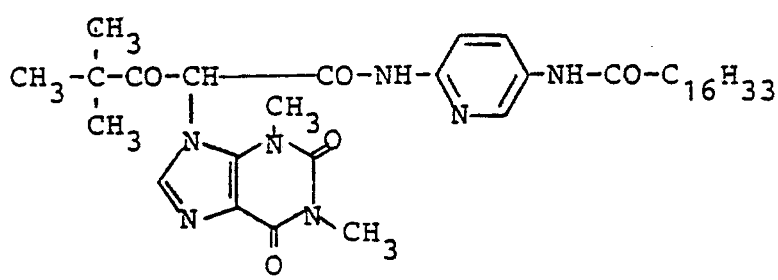

6 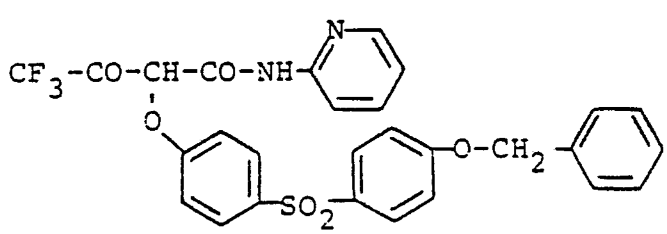

7 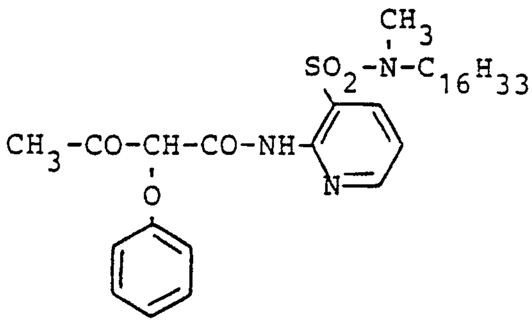

8 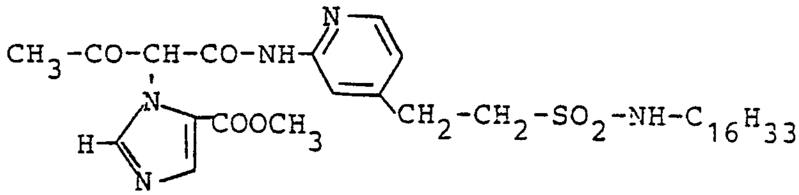

9 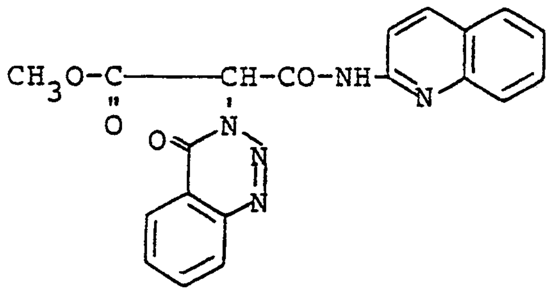

10 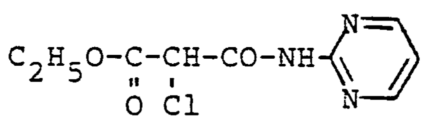

11 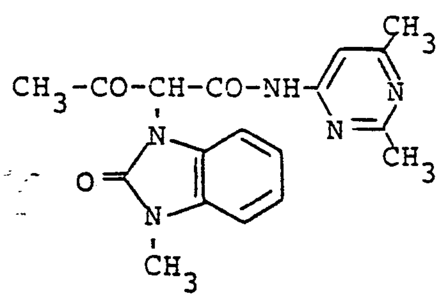

12 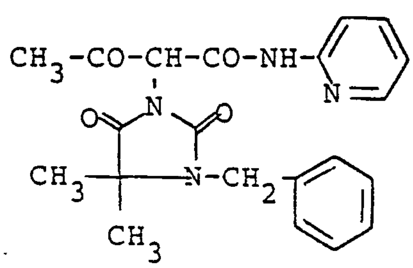

13 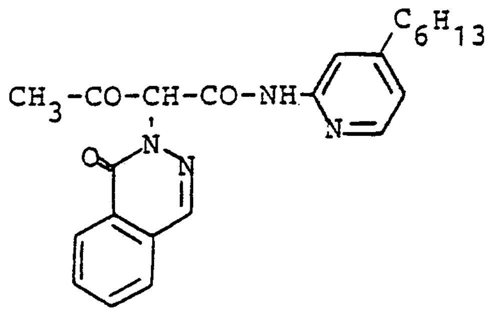

14 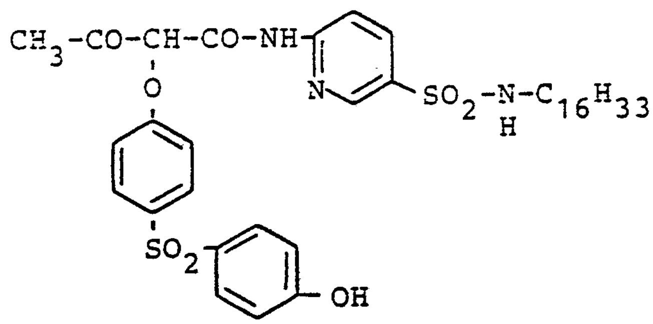

15 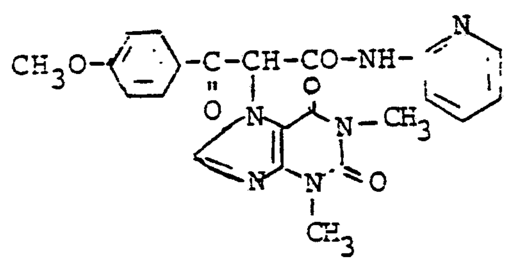

16 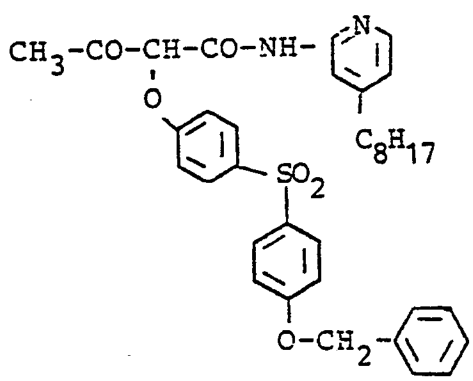

17 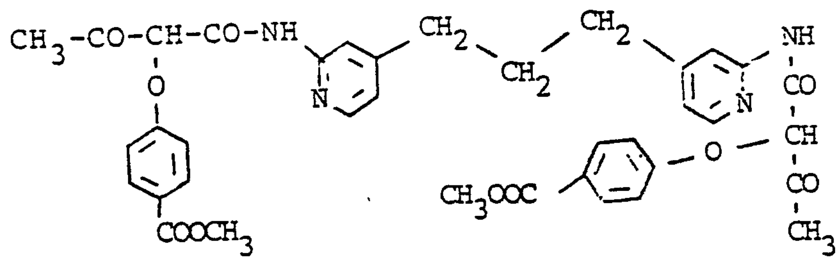

Die Synthese der erfindungsgemäßen Kuppler kann z.B. auf einem der folgenden Wege erfolgen:

1) 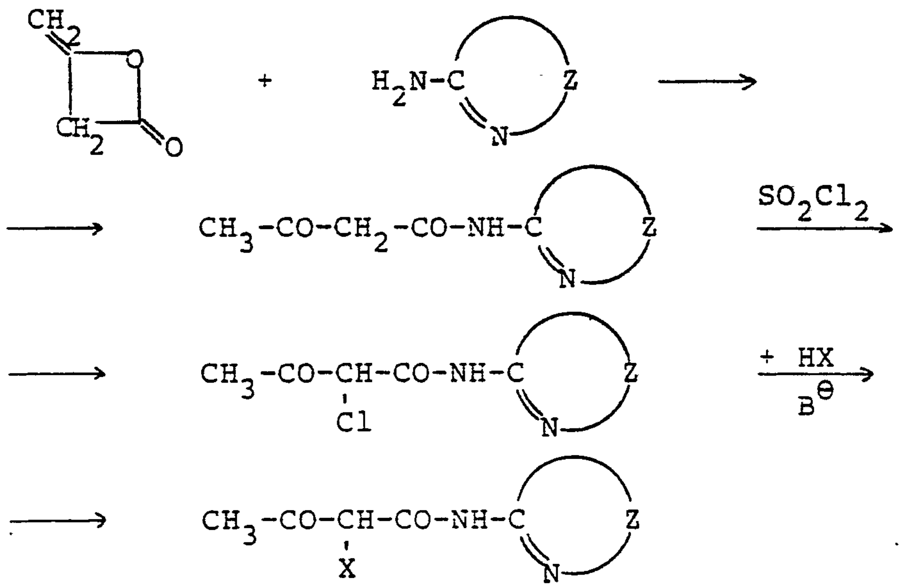

2) 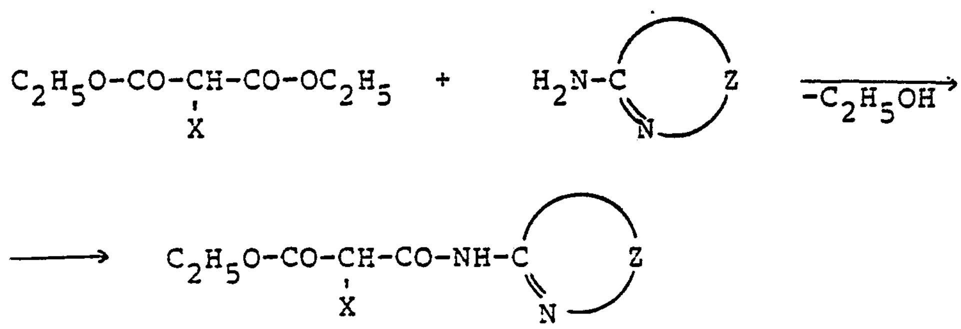


Die einzelnen Syntheseschritte verlaufen mit ausgezeichneten Ausbeuten und liefern Produkte von hervorragender Reinheit. Wenn auch im Einzelfall die Reaktionsbedingungen durch dem Durchschnittsfachmann geläufige Maßnahmen insbesondere bei der Einführung der abspaltbaren Gruppe optimiert werden müssen, so ist es doch überraschend, daß diese Einführung überhaupt möglich ist, ohne daß die gemäß nachfolgendem Formelschema zu erwartende spontane Cyclisierung eintritt.

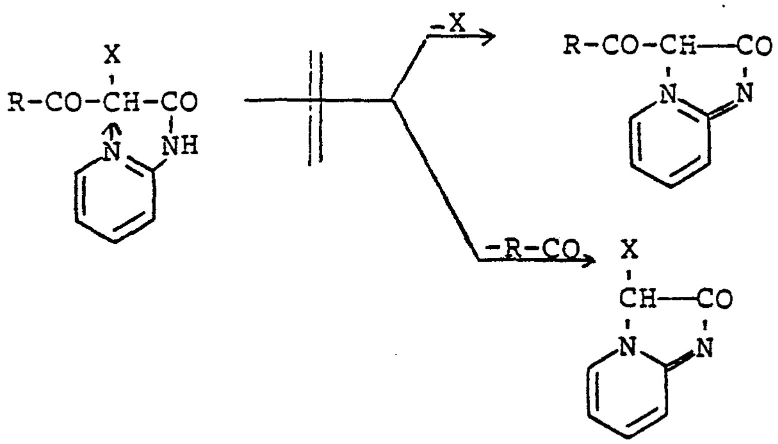

Herstellung von Kuppler Nr. 15

1) 94 g 2-Aminopyridin und
222 g 4-Methoxybenzoylessigester wurden in
600 ml Toluol zum Sieden erhitzt. Anschließend wurde 2 Stunden lang ein azeotropes Toluol/Wasser-Gemisch abdestilliert und und das Toluol durch frisches ersetzt. Die Reaktionsmischung wurde in
400 ml Chlorbutan eingerührt und einige Stunden bei Raumtemperatur stehen gelassen. Nach dem Absaugen wurde aus Chlorbutan umkristallisiert.
Schmelzpunkt: 120°C
Ausbeute: 192 g

2) 100 g 4-Methoxybenzoyl-acetaminopyridin wurden in
600 ml Methylenchlorid gelöst und bei -10°C mit
27,5 ml Sulfurylchlorid tropfenweise versetzt. Die Mischung wurde am Rotationsverdampfer im Vakuum schonend eingeengt und der Rückstand in wenig Methanol aufgenommen. Auf Zusatz von Ether schieden sich Kristalle ab, die nochmals aus Ethanol umkristallisiert wurden.
Schmelzpunkt: 156°C
Ausbeute: 52 g

3) 25 g α-Chlor-4-methoxybenzoyl-acetaminopyridin und

| | |
|---|---|
| 22 g | Theopyllin wurden in |
| 120 ml | Dimethylacetamid mit |
| 12 g | Na-methylat (95 %ig) auf 30°C erhitzt. |
| | Nach 2 Stunden wurde in |
| 1000 ml | Eis/Wasser eingerührt und wenig Essigsäure zugesetzt, bis sich Kristalle abschieden. Es wurde abgesaugt und mit wenig verdünnter Essigsäure nachgewaschen. Nach dem Trocknen wurde aus Essigester und anschließend aus Methanol umkristallisiert. |
| | Schmelzpunkt: 158°C |
| | Ausbeute: 26 g |

Die aus den erfindungsgemäßen Kupplern mit üblichen Farbentwicklern gebildeten Farbstoffe haben je nach Art des durch Z vervollständigten heterocyclischen Ringes und je nach Substitution ein Absorptionsmaximum bei ca. 550 nm und liegen damit im optimalen Spektralbereich für Purpurfarbstoffe.

Die gebildeten Farbstoffe zeigen eine ausgezeichnete Lichtstabilität und erweisen sich auch als hervorragend stabil in der Lagerung unter verschärften Umweltbedingungen, wie erhöhter Temperatur und/oder Feuchtigkeit.

Bemerkenswert ist vor allem die absolute Stabilität gegenüber Formalin, so daß auf alle zusätzlichen Maßnahmen zur Verbesserung der Formalinstabilität (z.B. Zusatz von Formalinfängern) verzichtet werden kann.

Die Reaktivität gegenüber 2-Äquivalentpurpurkupplern ist wesentlich erhöht.

Aufgrund der angegebenen Struktur besitzen die erfindungsgemäßen Kupplern durchweg gute Löslichkeiten, geringe Kristallisationstendenz und können demzufolge zu außergewöhnlich lagerstabilen Kupplerdispersionen verarbeitet werden.

Zur Verwendung in einem farbfotografischen Aufzeichnungsmaterial können die erfindungsgemäßen Kuppler nach einer der bekannten Methoden einer Silberhalogenidemulsion oder aber auch einem nicht lichtempfindlichen Bindemittelgemisch einverleibt werden. Soweit es sich bei den erfindungsgemäßen Kupplern um sogenannte Emulgierkuppler handelt, d.h. um hydrophobe Verbindungen, geschieht die Einarbeitung in bekannter Weise durch Lösen in geeigneten organischen Lösungsmitteln, z.B. in Estern aliphatischer Carbonsäuren, insbesondere in Essigestern, Diethylcarbonat, oder Methylenchlorid, und Einemulgieren dieser Lösung in eine gießfertige Silberhalogenidemulsion. Gegebenenfalls können hierbei in bekannter Weise auch sogenannte Ölbildner, das sind hochsiedende ölige Kupplerlösungsmittel, zur Anwendung gelangen.

Geeignete Ölbildner sind z.B. hochsiedende Säuren wie Dibenzylessigsäure oder Phenylethylessigsäure oder deren Ester, insbesondere Phthalsäureester, von

denen die Dialkylphthalate besonders geeignet sind, insbesondere Dibutylphthalat, hochsiedende Ketone wie Benzophenon oder Methyl-iso-butylketon oder Phosphorsäureester, beispielsweise Triarylphosphorsäureester wie z.B. Trikresylphosphat. Weitere Kupplerlösungsmittel sind beispielsweise beschrieben in US 2 322 027, US 3 689 271, US 3 764 336 und US 3 765 879.

Gemäß einer bevorzugten Ausführungsform geschieht das Einarbeiten der erfindungsgemäßen Farbkuppler in die Emulsion durch Lösen in vorzugsweise niedrig siedenden Lösungsmitteln wie Essigester, Diethylcarbonat, Methylenchlorid oder Alkylenchlorid unter Zusatz eines hochsiendenden Lösungsmittels wie z.B. Dibutylphthalat und Einemulgieren dieser Lösungen in die Silberhalogenidemulsion oder zunächst in wäßrige Gelatinelösung, worauf das erzeugte Emulgat einer Silberhalogenidemulsion zugesetzt werden kann.

Als lichtempfindliche Emulsionen eignen sich Emulsionen von Silberhalogeniden wie Silberchlorid, Silberbromid oder Gemischen davon, evtl. mit einem geringen Gehalt an Silberiodid bis zu 10 Mol-% in einem der üblicherweise verwendeten hydrophilen Bindemittel. Als Bindemittel für die fotografischen Schichten wird vorzugsweise Gelatine verwendet. Dies kann jedoch ganz oder teilweise durch andere natürliche oder synthetische

Bindemittel ersetzt werden. An natürlichen Bindemitteln sind z.B. Alginsäure und deren Derivate wie Salze, Ester oder Amide, Cellulose-Derivate wie Carboxymethylcellulose, Alkylcellulose wie Hydroxyethylcellulose, Stärke oder deren Derivate wie Ether oder Ester oder Caragenate geeignet. An synthetischen Bindemitteln seien erwähnt Polyvinylalkohol, teilweise verseiftes Polyvinylacetat, Polyvinylpyrrolidon und dergleichen.

Die Emulsionen können spektral sensibilisiert sein, z.B. durch die üblichen Mono- oder Polymethinfarbstoffe, wie saure oder basische Cyanine, Hemicyanine, Streptocyanine, Merocyanine, Oxonole, Hemioxonole, Styrylfarbstoffe oder andere, auch drei- oder mehrkernige Methinfarbstoffe, beispielsweise Rhodacyanine oder Neocyanine. Derartige Sensibilisatoren sind beispielsweise beschrieben in dem Werk von F.M. Hamer "The Cyanine Dyes and Related Compounds", (1964) Interscience Publishers John Wiley and Sons. Vorzugsweise werden die erfindungsgemäßen Kuppler in Kombination mit einer Silberhalogenidemulsion verwendet, die durch einen derartigen spektralen Sensibilisator für grünes Licht sensibilisiert ist.

Die Emulsionen können darüber hinaus in der üblichen Weise weitere Zusätze enthalten wie chemische Sensibilisatoren, Stabilisatoren, Entwicklungsbeschleuniger, Härtungsmittel, Antioxidantien, Weißkuppler und UV-Absorber.

Die Emulsionen können in der üblichen Weise gehärtet sein, beispielsweise mit Formaldehyd oder halogensubstituierten Aldehyden, die eine Carboxylgruppe enthalten, wie Mucobromsäure, Diketonen, Methansulfonsäureester, Dialdehyden und dergleichen.

Weiterhin können die fotografischen Schichten mit Härtern des Epoxityps, des heterocyclischen Ethyleniminytyps oder des Acryloyltyps gehärtet werden. Beispiele derartiger Härter sind z.B. in DE-OS 2 263 602 oder in GB 1 266 655 beschrieben. Weiterhin ist es auch möglich, die Schichten gemäß dem Verfahren der DE-OS 2 218 009 zu härten, um farbfotografische Materialien zu erzielen, die für eine Hochtemperaturverarbeitung geeignet sind.

Es ist ferner möglich, die fotografischen Schichten bzw. die farbfotografischen Mehrschichtenmaterialien mit Härtern der Diazin-, Triazin- oder 1,2-Dihydrochinolin-Reihe zu härten, wie in den GB-PSen 1 193 290, 1 251 091, 1 306 544, 1 266 655, der FR-PS 7 102 716 oder der DE-OS 23 32 317 beschrieben ist. Beispiele derartiger Härter sind Alkyl- oder Arylsulfonylgruppen-haltige Diazinderivate, Derivate von hydrierten Diazinen oder Triazinen, wie z.B. 1,3,5-Hexahydrotriazin, Fluor-substituierte Diazinderivate, wie z.B. Fluorpyrimidine, Ester von 2-substituierten 1,2-Dihydrochinolin- oder 1,2-Dihydroisochinolin-N-carbonsäuren. Brauchbar sind weiterhin Vinylsulfonsäurehärter, Carbodiimid- oder Carbamoylhärter, wie z.B. in

den DE-OSen 2 263 602, 2 225 230 und 1808 685, der FR-PS 1 491 807, der DE-PS 872 153 und der DDR-Patentschrift 7218 beschrieben. Weitere brauchbare Härter sind beispielsweise in der GB-PS 1 268 550 beschrieben.

Zur Erzeugung der Farbstoffe werden die gebräuchlichen Farbentwickler verwendet, z.B. die üblichen aromatischen, mindestens eine primäre Aminogruppe enthaltenden Verbindungen des p-Phenylendiamintyps.

Brauchbare Farbentwickler sind beispielsweise N,N-Dimethyl-p-phenylendiamin, N,N-Diethyl-p-phenylendiamin, 2-Amino-5-diethylaminotoluol, 2-Amino-5-(N-ethyl-N-hydroxyethylamino)-toluol, N-Butyl-N-   -sulfobutyl-p-phenylendiamin, 2-Amino-5-(N-ethyl-N-ß-methansulfonamidoethyl-amino)-toluol und dergleichen. Weitere brauchbare Farbentwickler sind beispielsweise beschrieben in J. Amer. Chem. Soc. 73, 3100 - 3125 (1951).

Die erfindungsgemäßen Materialien können beispielsweise Positiv-, Negativ- oder Umkehrmaterialien sein mit üblichen Schichtträgern, die in bekannter Weise zur Herstellung von fotografischen Materialien verwendet werden. Geeignet sind z.B. Folien aus Cellulosenitrat, Celluloseacetat, wie Cellulosetriacetat, Polystyrol, Polyester, wie Polyethylenterephthalat, Polyolefine, wie Polyethylen oder Polypropylen, eine barytierte oder eine polyolefin-kaschierte, wie z.B. eine polyethylenkaschierte Papierunterlage, sowie Glas und dergleichen.

Beispiel 1

Lösung A

Jeweils 1 m Mol der Kuppler 7,8 und 16 wurden in 5 ml Ethylacetat gelöst und zu jeder der erhaltenen Lösungen wurden 5 ml Dibutylphthalat hinzugegeben.

Lösung B

Netzmittel (Dodecylbenzolsulfonat, 1/10 der Gewichtsmenge an Kuppler) wurde in 37,5 g 7,5 %iger Gelatine-Lösung aufgelöst.

Emulgat A + B

Lösung B wurde in einem hochtourigen Rührgerät vorgelegt und bei 50-55°C wurde Lösung A darin emulgiert. Die Emulgierzeit betrug 3-5 min. Während dieser Zeit wurden Ethylacetatdämpfe abgesaugt. Auf diese Weise wurde von jedem der Kuppler 7, 8 und 16 ein Emulgat erhalten.

Emulsion C

Je 12,5 g Silberhalogenidemulsion (aus 70 g $AgNO_3$ mit 0,6 % AgI und 82,5 g Gelatine/kg Emulsion) wurden mit 0,6 mg Stabilisator (1-Phenyl-5-mercaptotetrazol), 120 mg Saponin und 80 mg Chromacetat versetzt und bei 40°C mit Emulgat A + B gemischt.

Die Gießlösungen wurden mit Wasser verdünnt und zu Schichten mit 5-6µm Dicke und einem Silberauftrag von 1,5 g $AgNO_3/m^2$ vergossen.

Zum Vergleich wurden in gleicher Weise Materialien mit folgenden Kupplern A, B und C hergestellt:

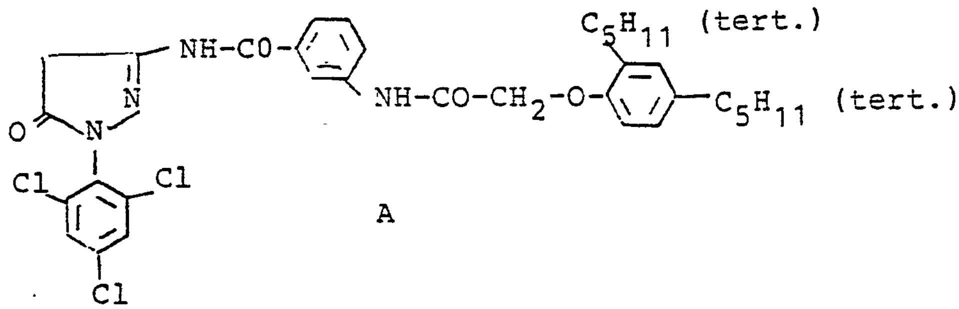

A

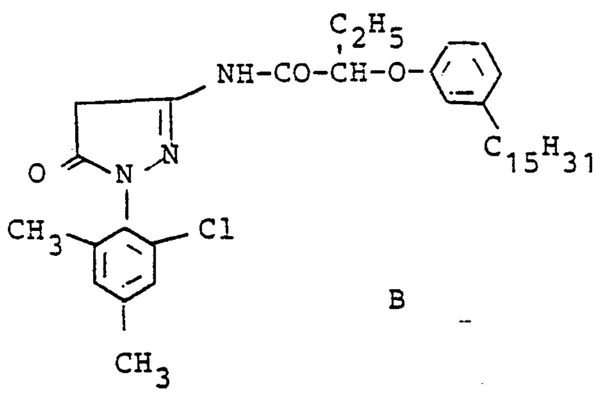

B

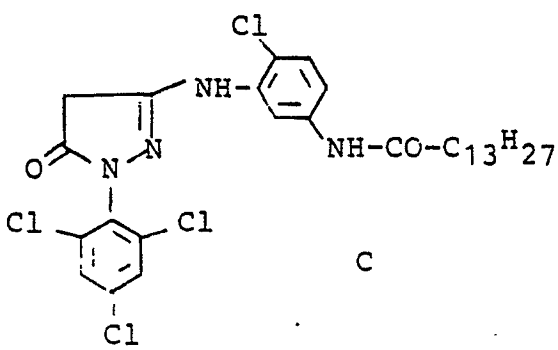

C

Je ein Streifen jedes der so erhaltenen Materialien wurde 16 Stunden lang einem Luftstrom (20°C; 50 - 60 % rel. Feuchte) ausgesetzt, der pro Liter 0,4 - 0,8 mg gasförmigen Formaldehyd enthielt.

Je ein weiterer Streifen der Materialien wurde mit einem entsprechenden Luftstrom ohne Formaldehyd behandelt.

Verarbeitung

Die Streifen wurden hinter einem Verlaufkeil belichtet und wie folgt verarbeitet:
Farbentwicklung 20°C/8 min; Wässerung 5 min; Bleichbad 5 min; Wässerung 5 min; Fixierbad 5 min; Wässerung 10 min.

Die Bäder hatten folgende Zusammensetzung:

Entwickler

| | | | |
|---|---|---|---|
| Teil A: | 400 | ml | $H_2O$ |
| | 2 | g | Polyphosphat |
| | 1,2 | g | Hydroxylaminchlorhydrat |
| | 5,75 | g | 3-Methyl-N-ethyl-N-methansulfonamidoethyl-p-phenylendiamin |

| | | | |
|---|---|---|---|
| Teil B: | 400 | ml | $H_2O$ |
| | 2 | g | Polyphosphat |
| | 2 | g | Natriumsulfit sicc. |
| | 75 | g | Kaliumcarbonat sicc. |
| | 2,5 | g | Kaliumbromid |

A langsam in B einrühren und mit Wasser auf 1 l auffüllen. Nach 12 h gebrauchsfertig; pH 10,8 - 11,0.

Bleichbad

| | | |
|---|---|---|
| 750 | ml | $H_2O$ |
| 3 | g | Na-Salz der Ethylendiamintetraessigsäure |
| 50 | g | Kaliumferricyanid |
| 15 | g | Kaliumbromid |
| 1 | g | Dinatriumphosphat |
| 19 | g | Monokaliumphosphat |

mit Wasser auf 1 l auffüllen; pH 5,3 - 5,7.

Fixierbad

| | | |
|---|---|---|
| 750 | ml | $H_2O$ |
| 200 | g | Natriumthiosulfat krist. |

mit Wasser auf 1 l auffüllen; pH 6 - 8.

Die nach der Entwicklung resultierenden Dichten sind in der folgenden Tabelle zusammengestellt:

Tabelle

| Kuppler | Dmax ohne Formaldehydbehandlung | Dmax mit Formaldehydbehandlung |
|---|---|---|
| 7 | 2,5 | 2,3 |
| 8 | 2,7 | 2,5 |
| 16 | 2,9 | 2,7 |
| A | 2,2 | 1,0 |
| B | 2,3 | 1,1 |
| C | 2,5 | 0,9 |

Aus den Ergebnissen der vorstehenden Tabelle geht hervor, daß die Materialien mit den Kupplern 7, 8 und 16 im Vergleich zu den Materialien mit den Kupplern A, B und C wesentlich weniger durch Formaldehyd geschädigt werden.

Beispiel 2

Die Absorptionsspektren der nach Beispiel 1 mit den Kupplern 8 und A erhaltenen und verarbeiteten Materialien wurden gemessen und miteinander verglichen. In Figur 1 sind die auf D = 1 normierten Absorptionskurven der Purpurfarbstoffe dargestellt, wobei die Kurve 1 dem Absorptionsspektrum des aus dem Kuppler 8 und die Kurve 2 demjenigen des aus dem Vergleichskuppler A

erhaltenen Bildfarbstoff entspricht. Die Spektren zeigen, daß die aus den erfindungsgemäßen Kupplern erzeugten Azomethinfarbstoffe hinsichtlich der Absorptionsspektren weitgehend mit demjenigen übereinstimmen, die aus üblichen Acylaminopyrazolonen erhalten werden.

Patentansprüche

1. Verfahren zur Herstellung eines Purpurbildes durch chromogene Entwicklung, bei dem ein bildmäßig belichtetes farbfotografisches Aufzeichnungsmaterial mit mindestens einer Silberhalogenidemulsionsschicht in Gegenwart einer Acylacetamidoverbindung und einer eine primäre Aminogruppe enthaltenden Farbentwicklerverbindung entwickelt wird, dadurch gekennzeichnet, daß die Entwicklung in Gegenwart einer Verbindung der folgenden Formel durchgeführt wird

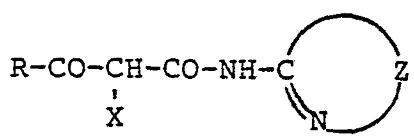

I

worin bedeuten

Z einen Rest zur Vervollständigung eines mindestens ein Stickstoffatom enthaltenden heterocyclischen Ringes,

X eine bei der chromogenen Entwicklung abspaltbare Gruppe des für 2-Äquivalentgelbkuppler üblichen Typs,

R Alkyl, Alkoxy, Aryl oder eine heterocyclisch aromatische Gruppe.

2. Verfahren nach Anspruch 1, dadurch gekennzeichnet, daß in Formel I Z den heterocyclischen Ring eines Pyridin-, 1,3-Diazin-, 1,3,5-Triazin-, Chinolin- oder Isochinolinringsystems vervollständigt.

3. Farbfotografisches Aufzeichnungsmaterial mit mindestens einer lichtempfindlichen Silberhalogenidemulsionsschicht und einer Acylacetamidoverbindung als Purpurkuppler, dadurch gekennzeichnet, daß die Acylacetamidoverbindung der folgenden Formel entspricht

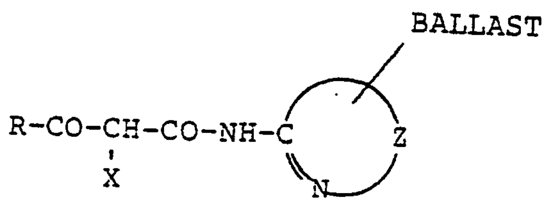

II

worin bedeuten

Z einen Rest zur Vervollständigung eines mindestens ein Stickstoffatom enthaltenden heterocyclischen Ringes,

X eine bei der chromogenen Entwicklung abspaltbare Gruppe des für 2-Äquivalentgelbkuppler üblichen Typs,

R Alkyl, Alkoxy, Aryl oder eine heterocyclisch aromatische Gruppe, und

BALLAST einen diffusionsfestmachenden Rest.

4. Material nach Anspruch 3, dadurch gekennzeichnet, daß in Formel II Z einen Pyridinring vervollständigt.

5. Material nach Anspruch 3, dadurch gekennzeichnet, daß in Formel II R Methyl, Trifluormethyl, Methoxy oder Ethoxy bedeutet.

6. Material nach Anspruch 3, dadurch gekennzeichnet, daß in Formel II X für die Gruppe $-OR^1$ steht, worin $R^1$ eine gegebenenfalls substituierte Phenylgruppe bedeutet.

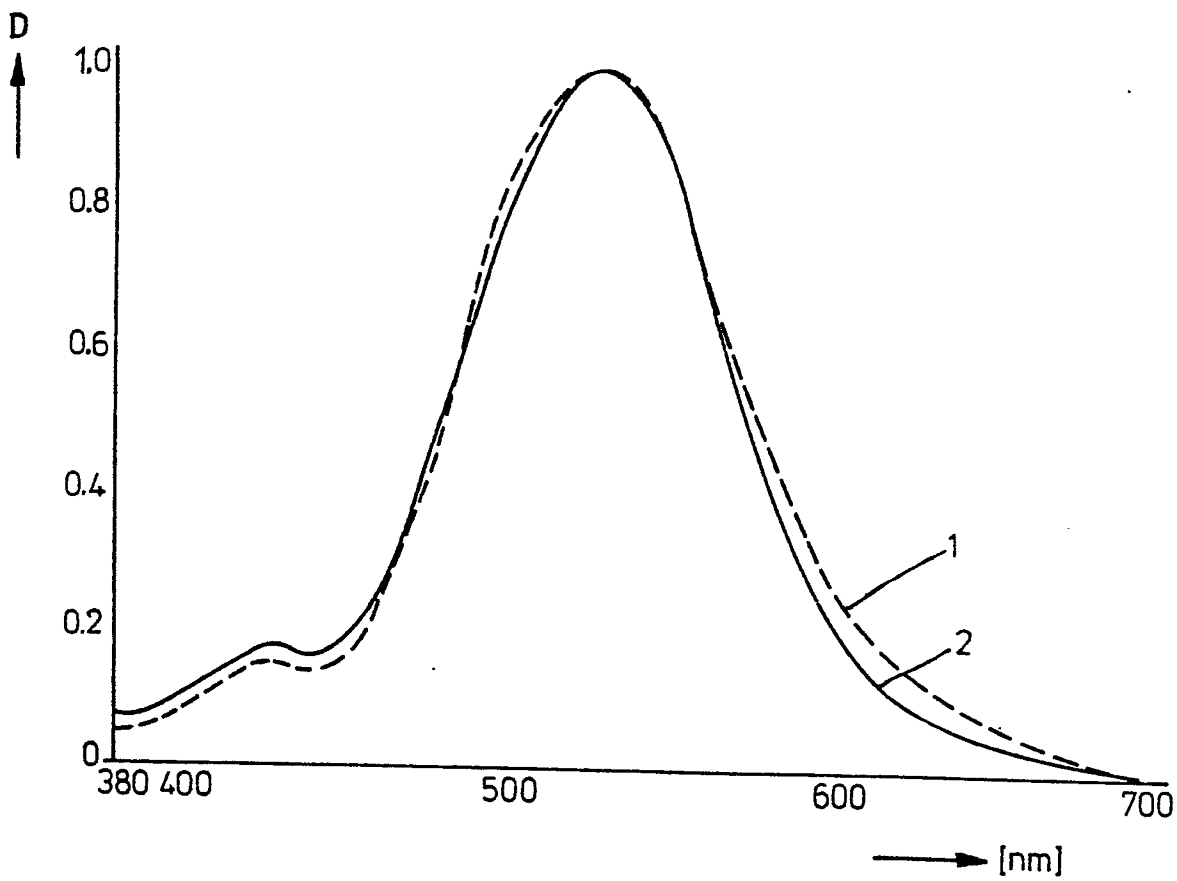


Fig.1

0066226

Europäisches Patentamt

# EUROPÄISCHER RECHERCHENBERICHT

Nummer der Anmeldung

EP 82104501.0

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D,X | DE - A1 - 2 528 860 (FUJI) * Patentansprüche 5,6; Seite 13, Zeile 14 - Seite 14, Zeile 17 * -- | 1,2 |
| D,X | DE - A - 2 263 875 (FUJI) * Patentanspruch 4; Seite 11, Zeile 23 - Seite 13, Zeile 9 * -- | 1,2 |
| X | DE - A1 - 2 840 381 (AGFA) * Seite 10, Zeilen 1-19 * ---- | 1,2 |

KLASSIFIKATION DER ANMELDUNG (Int. Cl. ³)

G 03 C 7/36
G 03 C 7/30
G 03 C 7/26
G 03 C 7/00

RECHERCHIERTE SACHGEBIETE (Int. Cl.³)

G 03 C

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 07-09-1982 | SCHÄFER |